# EUROPEAN PATENT APPLICATION

(11) **EP 3 085 693 A1**
(43) Date of publication of application: **26.10.2016**
(21) Application number: 15164351.7
(22) Date of filing: 20.04.2015
(51) Int. Cl.: C07D 219/02

(54) **NOVEL 3,6-DISUBSTITUTED FLUORENE DERIVATIVES**

(71) Applicant: Solvay SA, 1120 Bruxelles (BE)
(72) Inventor: Dabeux, Francois, 1140 Evere (BE); Bascour, Doinique, 1390 Grez-Doiceau (BE); Maunoury, Jonathan, 1030 Brussels (BE); Caille, Jean-Raphael, 5000 Namur (BE)
(74) Representative: Dr. Langfinger & Partner

(57) **Abstract**

Compounds of formula (1) 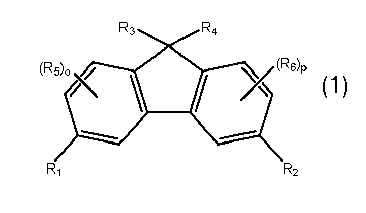 wherein R₁ and R₂, which may be the same or different, represent a substituent of formula

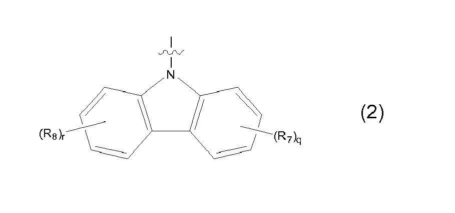

## Description

The present invention relates to novel 3,6-disubstituted fluorene derivatives, their use in organic electronic devices and organic electronic devices comprising the novel 3,6-disubstituted fluorene derivatives.

In organic electronic devices, the ability to form morphologically stable amorphous films is a key requirement for the development of small materials for organic light emitting diodes (OLEDs). That is because when a small molecule compound is used in the organic light-emitting layer, crystallization usually occurs if the molecule of the compound is too small and its structure is too symmetrical. Therefore, when applied in an organic emission layer, the small molecule compound is vulnerable to morphological change such as crystallization, and once the crystal is formed, it yields negative impacts upon the light-emitting nature and service life of the OLED.

Organic light-emitting diodes (OLEDs) exploit the property of materials of emitting light when they are excited by electrical current. OLEDs are of particular interest in this regard as an alternative to cathode ray tubes and to liquid-crystal displays for producing flat visual display units.

Due to the compact design and the intrinsically low power consumption compared to classical lighting devices, devices comprising OLEDs are suitable especially for mobile applications, for example for applications in mobile phones, lap-tops, digital cameras, and for illumination purposes.

The basic principles of the way in which OLEDs work and suitable structures (layers) of OLEDs are known to those skilled in the art.

The light-emitting materials (emitters) may be fluorescent materials (fluorescence emitters) or phosphorescent materials (phosphorescence emitters). The phosphorescence emitters have the advantage over fluorescent emitters that they exhibit triplet emission whereas fluorescence emitters only exhibit singlet emission. As a consequence, the quantum efficiency, energy efficiency and power efficiency of devices using phosphorescent emitters may be up to four times as high than for fluorescence emitters.

In order to implement the advantages of the use of the organometallic triplet emitters in practice, it is necessary to provide device compositions which have a high operative lifetime, a high stability to thermal stress and a low use and operating voltage.

Usually, organic light emitting devices comprise several layers to optimize the suitability of the device for the specific intended purpose.

The emissive layer comprises the emissive material and, in many cases a matrix material in which the emissive material is distributed. This material is generally referred to as host material.

Besides the emissive layer organic electronic devices usually comprise so called hole transport layers (HTL), sometimes also referred to as electron blocking layers.

Furthermore, additional layers may be present such as hole injection layers, electron injection layers etc.

For use in hole transport layers of hole injection layers as well as for the use as host materials in emissive layers, a high triplet level of the compounds is desirable. A high triplet level should avoid excitons quenching at the interface of hole transport layer and emissive layer and should thus improve the lifetime and efficiency of the devices.

Fluorene-based materials have been used in the active layer of thin film devices; most of the molecules which have been described in the literature are based on the fluorene-2,7-diyl structure.

Song et al., Tetrahedron Lett. 51(2010), 4894 describe the synthesis and properties of cyclic ethylene-bridged 3,6-fluorene dimers and linear analogues thereof.

US 2013/0341612 discloses 9,9'-(9,9-diphenyl-9H-fluorene-3,6-diyl)bis(9H-carbazole) of formula

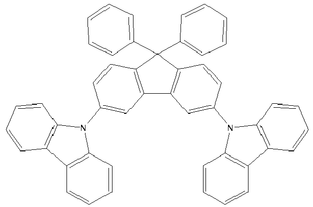

JP 2006/131782 discloses compounds 26-40 on pages 14 to 16 comprising a 9,9'-(9,9-diphenyl-9H-fluorene) as core bearing carbazolyl groups in 3 and 6 position (i.e. compounds having the same basic strucure as shown above) where the phenyl groups of the fluorene or the phenyl rings of the carbazolyl group bear various substituents.

JP 2010-114180 discloses compound PH-22 on page 15

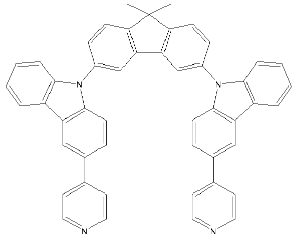

WO 2013/098175 discloses the following compounds on pages 8 and 9

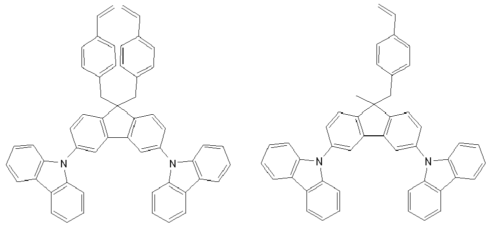

Phys. Chem. Chem. Phys. 12/47, 15448-15458 (2010) discloses the compound

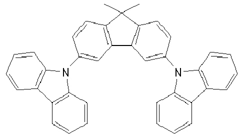

The compounds disclosed in the prior art referred to above are said to be useful in organic electronic devices.

While these prior art compounds have interesting properties, there still exists an ongoing need for additional compounds with high triplet level useful in organic electronic devices.

It was thus an object of the present invention to provide additional materials with high triplet level suitable for use in organic electronic devices, in particular in organic light emitting diodes.

This object has been achieved with the compounds in accordance with claim 1.

Preferred embodiments of the present invention are described in the dependent claims and the detailed specification hereinafter.

The compounds in accordance with the present invention are represented by formula (1)

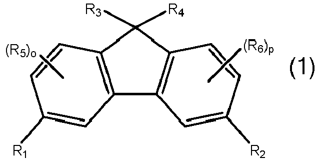

wherein R₁ and R₂, which may be the same or different, represent a substituent of formula (2)

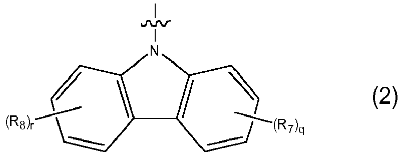

wherein
R₇ and R₈, which may be the same or different, represent hydrogen or an aryl, linear or branched alkyl, cycloalkyl, alkenyl, silyl, nitro, cyano or imino group,
o, p, q and r, which may be the same or different, are 0, 1, 2 or 3, preferably 0, 1 or 2, more preferably 0 or 1 and most preferably 0, R₃ and R₄, which may be the same or different, represent a group -(CH₂)_{n'}-Aryl, -(CH₂)ₙ-heteroaryl, (CH₂)ₙ-vinyl, -(CH₂)ₘ-Aryl-vinyl, or a cyano, nitro, silyl, C₃-C₁₈-alkyl, C₃-C₂₀-cycloalkyl, ether, thioether or polyether group, wherein n is 0 or an integer of from 1 to 20, preferably of from 1 to 8, n' is an integer of from 1 to 20, preferably of from 1 to 8
m is an integer of from 3 to 20, Aryl is a C₅-C₃₀ aryl group and heteroaryl is a C₂-C₃₀ heteroaryl group, and
R₅ and R₆, which may be the same or different, represent a substituent other than hydrogen.

If R₅ and or R₆ are present, they are preferably selected from groups as defined above for R₇ and R₈ other than hydrogen. In accordance with a preferred embodiment, o and p are 0, i.e. no substituents R₅ and R₆ are present in the respective rings.

Compounds in accordance with claim 1 or 2 wherein R₇ and R₈, which may be the same or different, represent a C₃-C₈-alkyl group are also preferred.

Another preferred class of compounds of formula (1) are those, in which R₃ and R₄ wherein R₃ and R₄, which may be the same or different are represented by a -(CH₂)ₘ-Aryl-Vinyl group, in which Aryl is particularly preferred a phenyl group. m is preferably an integer of from 3 to 12, particularly preferred of from 3 to 8.

Compounds in which R₃ and R₄ represent an alkyl group are also preferred.

The term aryl, as used herein, shall mean a C₅-C₃₀ aryl group and for the purposes of the present invention denotes groups having at least one 5 to 7 membered aromatic ring with (4n+2) *π* electrons in accordance with the so called Hückel rule. Two or more aromatic rings may be annealed of fused or may be connected through a bond or an alkyl group with each other.

Representative aryl groups are thus phenyl, biphenyl, naphthyl or anthracenyl.

The aromatic rings in the aryl groups may be un-substituted or substituted by substituents selected from the group consisting of halogen, alkyl, alkoxy, amino, cyano, alkenyl, alkynyl, arylalkyl, aryl and heteroaryl groups.

Particularly preferred aryl groups are C₆-C₁₄ aryl groups like phenyl, biphenyl naphthyl and anthracenyl, which may be substituted or unsubstituted and in some cases substituted or unsubstituted phenyl, in particular unsubstituted phenyl have shown advantageous results.

The term alkyl group, as used herein, denotes a linear, branched or cyclic group comprising 3 to 18 carbon atoms with hydrogen atoms as substituents. Linear or branched alkyl groups having 3 to 12, in particular 3 to 8 carbon atoms are preferred. Linear alkyl groups having 3 to 6 carbon atoms such as n-propyl, n-butyl, n-pentyl and n-hexyl are especially preferred.

Representative examples of branched alkyl groups with 3 to 8 carbon atoms are i-propyl, i- and t-butyl and the isomeric branched methyl-or ethyl-substituted pentyl- or hexyl derivatives like e.g. 2-methylpentyl, 3-methylpentyl, 2-ethylhexyl, 3-ethylhexyl or 4-ethylhexyl to mention only a few typical representatives.

The term alkenyl group, as used herein, shall mean substituted or unsubstituted linear or branched aliphatic groups with 2 to 18 carbon atoms and at least one carbon-carbon double bond in the main chain. A preferred alkenyl group is the ethenyl group -CH=CH₂ which is also commonly designated as vinyl group. A further preferred group is the 1-propenyl or allyl group -CH₂-CH=CH₂.

The term heteroaryl group, when used herein, denotes a group with 2 to 30 carbon atoms having an aromatic ring comprising at least one heteroatom, which may be substituted or unsubstituted as described above for the aryl groups.

Preferred heteroaryl groups are heteroaryl rings containing at least one donor nitrogen atom. Said rings may be un-substituted or substituted by substituents selected from the group consisting of halogen, alkyl, alkoxy, amino, cyano, alkenyl, alkynyl, arylalkyl, aryl and heteroaryl group and/or may form an annealed ring system with other rings selected from cycloalkyl, aryl and heteroaryl rings. Heteroaryl substituents may be preferably un-substituted or substituted carbazolyl or un-substituted or substituted dibenzofuranyl.

More particularly heteroaryl groups are derived from the heteroarenes group consisting of 2H-pyrrole, 3H-pyrrole, 1H-imidazole, 2H-imidazole, 4H-imidazole,1H-1,2,3-triazole, 2H-1,2,3-triazole, 1H-1,2,4-triazole, 1H-pyrazole, 1H-1,2,3,4-tetrazole, imidazol-2-ylidene, oxazole, isoxazole, thiazole, isothiazole, 1,2,3-oxadiazole, 1,2,5-oxadiazole, 1,2,3- thiadiazole and 1,2,5-thiadazole rings.

Nitrogen containing heteroaryl groups are preferably derived from the heteroarenes shown below

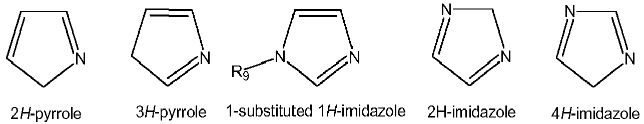

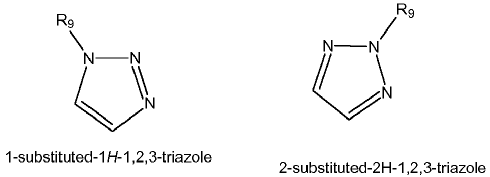

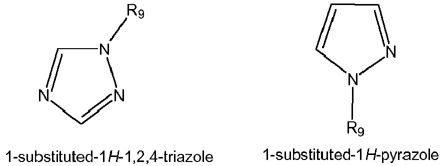

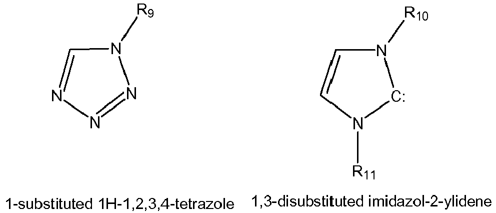

wherein R₉, R₁₀ and R₁₁ may be selected from a broad variety of substituents such as alkyl, alkenyl, alkynyl, arylalkyl, aryl and heteroaryl groups.

Further preferred heteroaryl groups are derived from the following heteroarenes

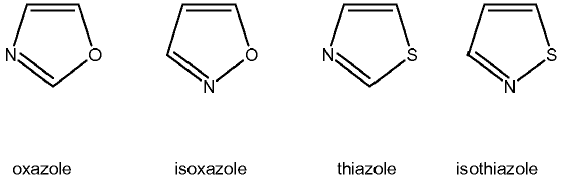

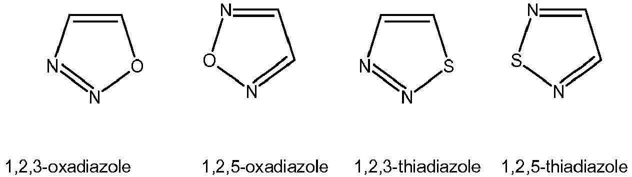

wherein the rings can be unsubstituted or substituted with one or more substituents.

Still another preferred group of heteroaryl substituents comprises the 6-membered ring systems shown below:

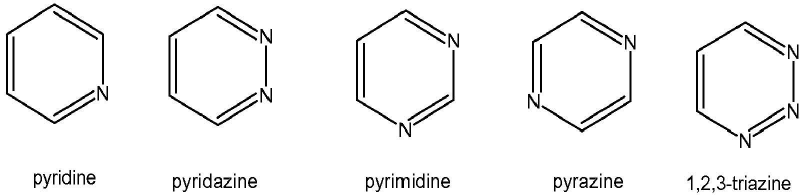

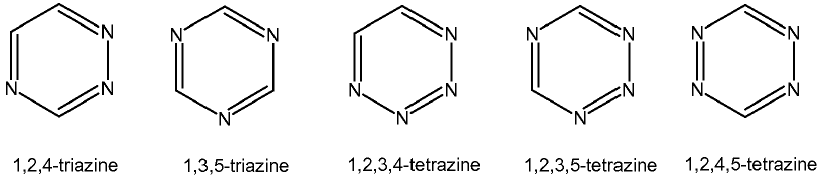

The heteroaryl groups may form or be part of an annealed ring system where several rings are condensed or annealed.

The term cycloalkyl, as used herein, is used for a carbocyclic group with 3 to 20 carbon atoms, which may be substituted or unsubstituted. Representative examples are substituted or unsubstituted cycloalkyl groups with 5 to 6 carbon atoms and preferably 5 to 7 carbon atoms in the ring.

The optional substituents of the aforementioned aryl, heteroaryl or cycloalkyl groups, are preferably selected from aliphatic groups, carbocyclic groups, aromatic groups or heterocyclic groups, oxo, OR¹², NR¹³R¹⁴ and SR¹⁵ groups, wherein R¹² to R¹⁵ are the same or different and represent hydrogen, an aliphatic group, a carbocyclic group, an aromatic group or a heterocyclic group having 1 to 20 carbon atoms.

In accordance with still another preferred embodiment, the substituents of the aliphatic, carbocyclic, aromatic or heteroaromatic groups are selected from the group consisting of halogen, alkyl, alkoxy, aryloxy, oxo, amino, substituted amino, cyano, alkenyl, alkynyl, arylalkyl, aryl and heteroaryl groups, even more preferably from halogen, alkyl, alkoxy, amino, cyano, alkenyl, alkynyl, arylalkyl, aryl and heteroaryl groups.

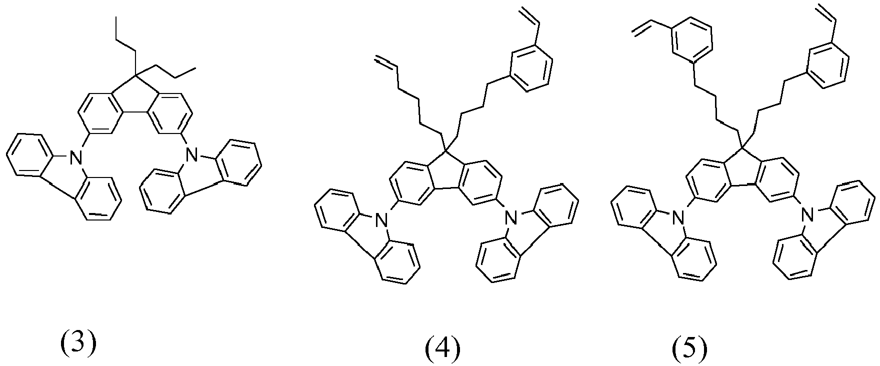

The variables o, p, q and r, which may be the same or different, are each 0, 1, 2 or 3, preferably 0, 1 or 2 and in some cases compounds in which o, p, q and r are 0 have shown advantageous properties.

Compounds (3) to (5) shown below represent a group of particularly preferred compounds

The compounds in accordance with the present invention usually have a triplet level exceeding 2.8 eV and are therefore particularly suitable for use in organic electronic devices, in particular organic light emitting diode (OLED).

Accordingly, another embodiment of the present invention is directed to organic electronic devices, in particular organic light emitting diodes, comprising a compound in accordance with the present invention, preferably in the hole transport layer, the hole injection layer or as a host material in the emissive layer.

An OLED generally comprises :
a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);
an electron injection layer (EIL) such as LiF, Cs₂CO₃
a cathode, generally a metallic cathode, such as an Al layer.

For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).
Due to their high triplet level, the compounds of the present invention may help to avoid exciton quenching at the interface e.g. of HTL and EML which improves efficiency and lifetime of the devices.

The compounds in accordance with the present invention may be synthesized using processes known to the skilled person, who will select the appropriate process, reactants and reactant conditions based on his professional knowledge and adopted to the specific compound to be synthesized.

Generally said, the compounds of the present invention , from a retrosynthesis point of view, can be synthesized from a common precursor, 3,6-dibromofluorenone, which is commercially available or may be synthesized in a manner known to the skilled person.

Generally, 3,6-dibromofluorenone is reduced to its fluorene analogue before introduction of substituents R₃ and R₄ by a suitable reagent. A subsequent Buchwald-Hartwig coupling with the respective reactants providing groups R₁ and R₂ then yields the final product.

An exemplary reaction scheme for the synthesis of preferred compounds is the following:

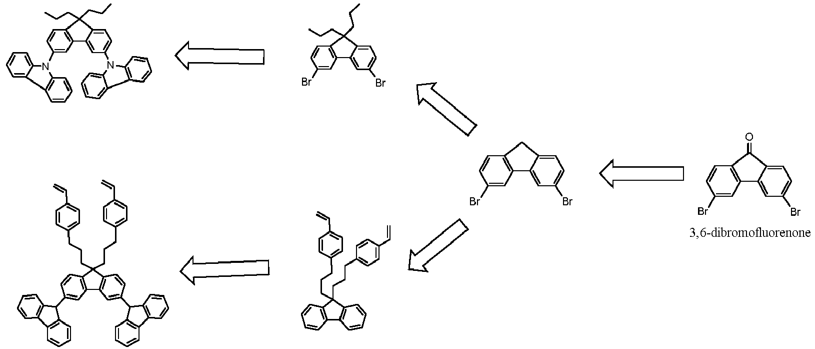

The reduction of the dibromofluorenone in accordance with the following exemplary scheme

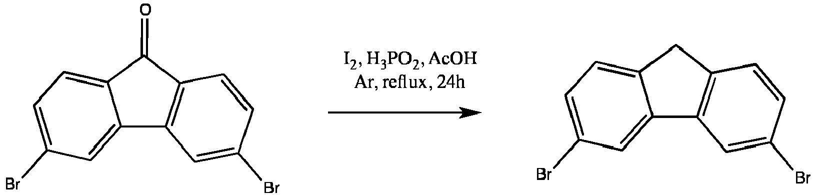

has been extensively described in the literature. Traditional methods are well known as Wolf-Kishner and Clemmensen reductions and suitableconditions are i.a.described by Hicks and coworkers (Hicks, L. ; Han, J. K. ; Fry, A. J. ; Tetrahedron Lett., 2000, 41, 7817-7820) to which reference is made for further details.

Introducing substituents R₃ and R₄ is exemplary shown in the following scheme for alkyl groups, which represent a preferred group of substituents in the compounds in accordance with the present invention.

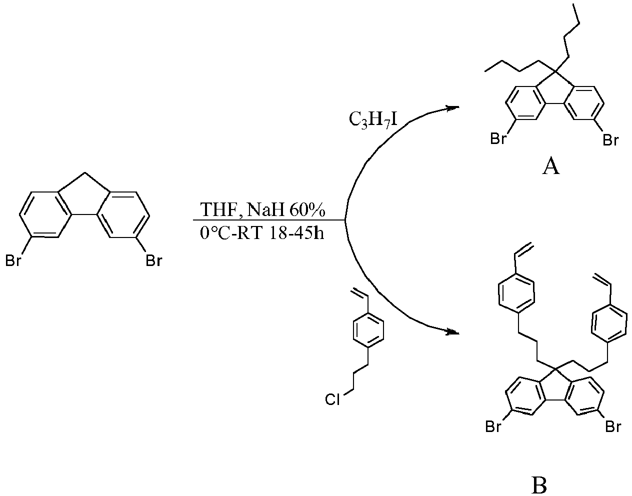

The final step is then preferably a so-called Buchwald-Hartwig coupling or other suitable coupling method with which substituents R₁ and R₂ are introduced to obtain the desired product.

Suitable carbon-carbon coupling methods have been described extensively in the literature and are known to the skilled person so that no further details need to be given here.

## Claims

1. Compounds of formula (1) 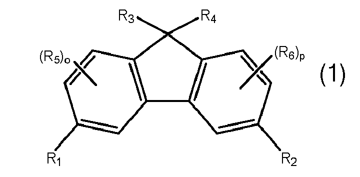 wherein R₁ and R₂, which may be the same or different, represent a 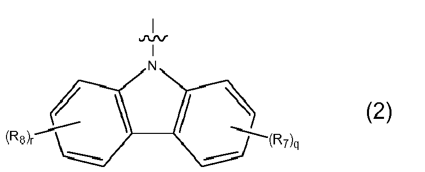 substituent of formula (2)
wherein
R₇ and R₈, which may be the same or different, represent hydrogen or an aryl, linear or branched alkyl, cycloalkyl, alkenyl, silyl, nitro, cyano or imino group, o, p, q and r, which may be the same or different, are 0, 1, 2 or 3,
R₃ and R₄, which may be the same or different, represent a group -(CH₂)_{n'}-Aryl, -(CH₂)ₙ-heteroaryl, (CH₂)_{n'}-vinyl, -(CH₂)ₘ-Aryl-vinyl, or a cyano, nitro, silyl, C₃-C₁₈-alkyl, C₃-C₂₀-cycloalkyl, ether, thioether or polyether group, wherein n is 0 or an integer of from 1 to 20, preferably of from 1 to 8, n' is an integer of from 1 to 20, m is an integer of from 3 to 20, Aryl is a C₅-C₃₀ aryl group and heteroaryl is a C₂-C₃₀ heteroaryl group, and
R₅ and R₆, which may be the same or different, represent a substituent other than hydrogen.

2. Compounds in accordance with claim 1 or 2 wherein R₇ and R₈, which may be the same or different, represent a C₁-C₈-alkyl group.

3. Compounds in accordance with any of claims 1 to 4 wherein R₃ and R₄, which may be the same or different are represented by a -(CH₂)ₘ-Aryl-Vinyl group.

4. Compounds in accordance with claim 5 wherein the aryl group is a phenyl group.

5. Compounds in accordance with any of claims 1 to 4 wherein R₃ and R₄, which may be the same or different are represented by an alkyl group.

6. Compounds in accordance with claim 7 wherein the alkyl group is a propyl or butyl group.

7. Compounds of formula (3) to (5)

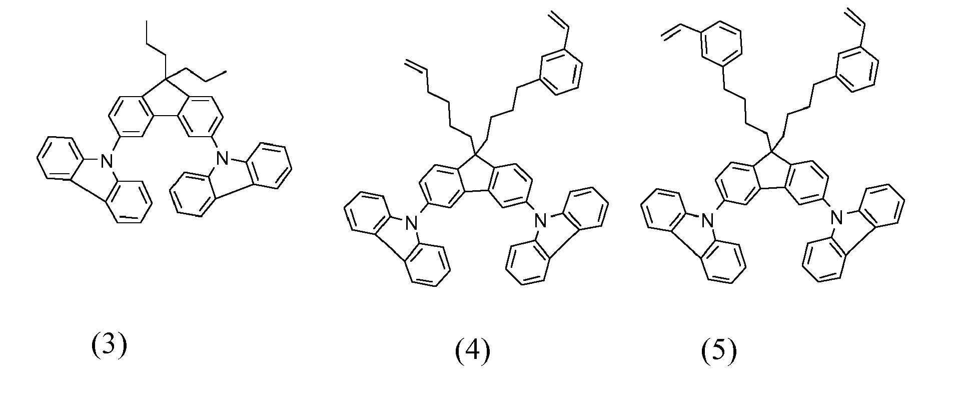

8. Use of the compounds in accordance with any of claims 1 to 7 in organic electronic devices.

9. Use in accordance with claim 8 wherein the organic electronic device is an organic light emitting diode (OLED).

10. Organic electronic device comprising a compound in accordance with any of claims 1 to 7.

11. Organic electronic device in accordance with claim 10 which is an organic light emitting diode (OLED).

12. Organic light emitting diode in accordance with claim 11 comprising a compound in accordance with any of claims 1 to 7 in the hole transport layer, and/or the hole injection layer.

13. Organic light emitting diode in accordance with claim 11 comprising a compound in accordance with any of claims 1 to 7 as host material in an emissive layer.
